# EUROPÄISCHE PATENTANMELDUNG

(11) **EP 1 182 197 A1**
(43) Veröffentlichungstag der Anmeldung: **27.02.2002**
(21) Anmeldenummer: 00118442.3
(22) Anmeldetag: 24.08.2000
(51) Int. Cl.: C07D 233/54, C07D 213/20, C07C 211/63, C07C 309/73

(54) **Einstufiges Verfahren zur Darstellung ionischer Flüssigkeiten**

(71) Anmelder: Solvent Innovation GmbH, 50679 Köln (DE)
(72) Erfinder: Wasserscheid, Peter, 50829 Köln (DE); Hilgers, Claus, 50679 Köln (DE); Boesmann, Andreas, 52068 Aachen (DE)
(74) Vertreter: Weber, Thomas, Dr. Dipl.-Chem.

(57) **Zusammenfassung**

Die Erfindung betrifft ein einstufiges Verfahren zur Herstellung ionischer Flüssigkeiten der allgemeinen Formel

[A]ₙ⁺[Y]ⁿ⁻, (n=1 oder 2)

durch Alkylierung der zugrundeliegenden Amine, Phosphine, Imidazole, Pyrazole, Triazole oder Pyridine mit einem Halogenid RX und Austausch des Halogenidanions X durch das Anion [Y]⁻ oder [Y]²⁻ ohne Isolierung der Zwischenprodukte.

## Beschreibung

Die Erfindung betrifft ein einstufiges Verfahren zur Herstellung ionischer Flüssigkeiten der allgemeinen Formel

[A]ₙ⁺ [Y]ⁿ⁻,

durch Alkylierung der zugrundeliegenden Amine, Phosphine, Imidazole, Pyrazole, Triazole oder Pyridine mit einem Halogenid RX und Austausch des Halogenidanions X⁻ durch das Anion [Y]⁻ oder [Y]²⁻ ohne Isolierung der Zwischenprodukte.

Unter ionischen Flüssigkeiten versteht man Salze oder Gemische aus Salzen, deren Schmelzpunkte unterhalb 80°C liegen. Diese Salze bestehen aus Anionen wie z.B. Halogenostannaten, Halogenoaluminaten, Hexafluorophosphaten oder Tetrafluoroboraten kombiniert mit substituierten Ammonium-, Phosphonium, Pyridinium- oder Imidazolium-Kationen. Mehrere Veröffentlichungen beschreiben bereits die Verwendung ionischer Flüssigkeiten als Lösungsmittel für Übergangsmetall-katalysierte Reaktionen (Übersichtsartikel: T. Welton, *Chem*. *Rev.* **1999,** *99*, 2071) Beispielsweise wurden Hydrierungen von Olefinen mit Rhodium(I) ) (P. A. Z. Suarez, J. E. L. Dullius, S. Einloft, R. F. de Souza und J. Dupont, *Polyhedron 15/7,* **1996,** 1217-1219), Ruthenium(II) und Cobalt(II) komplexen (P. A. Z. Suarez, J. E. L. Dullius, S. Einloft, R. F. de Souza und J. Dupont, *Inorganica Chimica Acta 255,* **1997,** 207-209) in ionischen Flüssigkeiten mit Tetrafluoroborat-Anion erfolgreich bearbeitet. Auch die Hydroformylierung von funktionalisierten und unfunktionalisierten Olefinen gelingt mit Rhodium-Katalysatoren in ionischen Flüssigkeiten mit schwach koordinierenden Anionen (z.B. PF₆⁻, BF₄⁻) EP-A-0776880, Y. Chauvin, L. Mussmann, H. Olivier, *Angew*. *Chem*., *Int*. *Ed*. *Engl*., **1995,** *34,* 2698; W. Keim, D. Vogt, H. Waffenschmidt, P. Wasserscheid, *J. of Cat.,* **1999,** *186,* 481).

Zur Synthese von binären ionischen Flüssigkeiten vom Typ [A]⁺[Y]⁻ wird bisher ein zweistufiges Verfahren angewendet (J. S. Wilkes, M. J. Zaworotko, *J. Chem. Soc., Chem. Commun.,* 13, **1992,** 965). Dabei wird zunächst durch Reaktion eines Alkylierungsreagenz RX und eines Amins NR¹R²R³ oder eines Phosphans PR¹R²R³ in einer Quarternisierungsreaktion das organische Ammoniumsalz [NR¹R²R³R]⁺ X⁻ oder das organische Phosphoniumsalz [PR¹R²R³R]⁺ X⁻ aufgebaut. X⁻ ist dabei in der Regel ein Halogenidion. Das organische Halogenidsalz wird isoliert und in einer nachfolgenden, zweiten Reaktionsstufe in einer Austauschreaktion mit dem Alkali- oder Erdalkalisalz des Typs M⁺ [Y]⁻ umgesetzt. Dies geschieht in einem Lösungsmittel, in dem das Nebenprodukt M⁺ X⁻ schwerlöslich, die zu synthetisierende ionische Flüssigkeit [A]⁺[Y]⁻ dagegen gut löslich ist.

Dieses zweistufige Verfahren wurde in der Literatur zur Darstellung von ionischen Flüssigkeiten mit [BF₄]⁻-, [PF₆]⁻-, Acetat-, Nitrat-, HSO₄⁻-, SO₄²⁻-Ionen erfolgreich verwendet (J. S. Wilkes, M. J. Zaorotko, *J. Chem. Soc*., *Chem. Commun.,* 13, **1992,** 965, B. Ellis, *WO 9618459 A1 960620,* **1996** J. Fuller, R. T. Carlin, H. C. de Long, D. Haworth, *J*. *Chem*. *Soc*., *Chem*. *Commun*., 3, **1994,** 299).

Von Nachteil ist bei dieser Reaktionsführung, dass nur dann ein quantitativer Austausch des Halogenidsalzes [NR¹R²R³R]⁺ X⁻ bzw. [PR¹R²R³R]⁺ X⁻ zur gewünschten ionischen Flüssigkeit [NR¹R²R³R]⁺ [Y]⁻ bzw. [PR¹R²R³R]⁺ [Y]⁻ gelingt, wenn unter Austauschbedingungen das Reaktionssystem vollständig wasserfrei ist. Dies ist von entscheidender Bedeutung für die Produktqualität, da Wasserspuren im Reaktionssystem unter Austauschbedingungen unweigerlich zu Verunreinigungen des Produkts mit Halogenidionen führen. Da Halogenidionen für zahlreiche Übergangsmetallkatalysatoren starke Katalysatorgifte darstellen, ist die Reinheit der hergestellten ionischen Flüssigkeiten für viele Anwendungen das entscheidende Qualitätskriterium.

Für die Produktion binärer ionischer Flüssigkeiten vom Typ [A]⁺[Y]⁻ birgt das literaturbekannte und bisher ausschließlich praktizierte, zweistufige Verfahren zwei wesentliche Nachteile, die vor allem bei der Produktion größerer Mengen von großer Bedeutung sind:
1) Das zunächst dargestellte Halogenidsalz ist stark hygroskopisch d. h. um Wasserspuren im Halogenidsalz im Hinblick auf die nachfolgende Austauschreaktion zu vermeiden muß die Isolierung (Filtration) des Halogenidsalzes unter Inertbedingungen erfolgen. Dieser Schritt ist besonders zeitraubend und aufwendig, weil in der Regel die Halogenidsalze bemerkenswert schlecht mechanisch zerkleinert und umgefüllt werden können.
2) Die Austauschreaktion selbst erfordert ebenfalls ein absolut wasserfreies Lösungsmittel. Das Trocknen und Absolutieren des Lösungsmittels für die Austauschreaktion ist ein langwieriger und kostenintensiver Prozess.

Aufgabe der vorliegenden Erfindung war es dementsprechend ein Verfahren zur Herstellung ionischer Flüssigkeiten zu Verfügung zu stellen, welches die oben genannten Nachteile nicht aufweist und zu Produkten führt, welche in hoher Reinheit und Ausbeute als unmittelbares Verfahrensprodukt zur Verfügung stehen, und zudem ein leichtes Scale-Up ermöglicht.

Die Aufgabe wurde erfindungsgemäß gelöst durch ein Verfahren zur Herstellung ionischer Flüssigkeiten der allgemeinen Formel

[A]ₙ⁺[Y]ⁿ⁻,

wobei n = 1 oder 2 ist und
das Anion [Y]⁻ ausgewählt ist aus der Gruppe bestehend aus Tetrafluoroborat ([BF₄]⁻), Tetrachloroborat ([BCl₄]⁻), Hexafluorophosphat ([PF₆]⁻), Hexafluoroantimonat ([SbF₆]⁻), Hexafluoroarsenat ([AsF₆]⁻ ), Tetrachloroaluminat ([AlCl₄]⁻), Trichlorozinkat [(ZnCl₃]⁻), Dichlorocuprat ([CuCl₂]⁻),Sulfat ([SO₄]²⁻), Carbonat ([CO₃]²⁻), Fluorosulfonat, [R'-COO]⁻, [R'-SO₃]⁻ oder [(R'-SO₂)₂N]⁻, und R' ein linearer oder verzweigter 1 bis 12 Kohlenstoffatome enthaltender aliphatischer oder alicyclischer Alkyl- oder ein C₅-C₁₈-Aryl-, C₅-C₁₈-Aryl-C₁-C₆-alkyl- oder C₁-C₆-Alkyl-C₅-C₁₈-aryl-Rest ist, der durch Halogenatome substituiert sein kann,
das Kation [A]⁺ ist ausgewählt aus
- quarternären Ammonium-Kationen der allgemeinen Formel

   [NR¹R²R³R]⁺,
- Phosphonium-Kationen der allgemeinen Formel

   [PR¹R²R³R]⁺,
- Imidazolium-Kationen der allgemeinen Formel 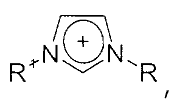 wobei der Imidazol-Kern substituiert sein kann mit wenigstens einer Gruppe, die ausgewählt ist aus C₁-C₆-Alkyl-, C₁-C₆-Alkoxy-, C₁-C₆-Aminoalkyl-, C₅-C₁₂-Aryl- oder C₅-C₁₂-Aryl-C₁-C₆-Alkylgruppen,
- Pyridinium-Kationen der allgemeinen Formel 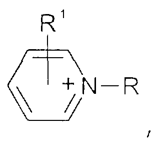 wobei der Pyridin-Kern substituiert sein kann mit mit wenigstens einer Gruppe, die ausgewählt ist aus C₁-C₆-Alkyl-, C₁-C₆-Alkoxy-, C₁-C₆-Aminoalkyl-, C₅-C₁₂-Aryl- oder C₅-C₁₂-Aryl-C₁-C₆-Alkylgruppen,
- Pyrazolium-Kationen der allgemeinen Formel 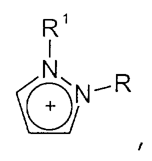 wobei der Pyrazol-Kern substituiert sein kann mit mit wenigstens einer Gruppe, die ausgewählt ist aus C₁-C₆-Alkyl-, C₁-C₆-Alkoxy-, C₁-C₆-Aminoalkyl-, C₅-C₁₂-Aryl- oder C₅-C₁₂-Aryl-C₁-C₆-Alkylgruppen,
- und Triazolium-Kationen der allgemeinen Formel 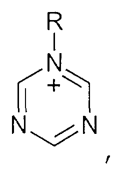 wobei der Triazol-Kern substituiert sein kann mit mit wenigstens einer Gruppe, die ausgewählt ist aus C₁-C₆-Alkyl-, C₁-C₆-Alkoxy-, C₁-C₆-Aminoalkyl-, C₅-C₁₂-Aryl- oder C₅-C₁₂-Aryl-C₁-C₆-Alkylgruppen,
und die Reste R¹, R², R³ unabhängig voneinander ausgewählt sind aus der Gruppe bestehend aus
(i) Wasserstoff;
(ii) linearen oder verzweigten, gesättigten oder ungesättigten, aliphatischen oder alicyclischen Alkylgruppen mit 1 bis 20 Kohlenstoffatomen;
(iii) Heteroaryl-, Heteroaryl-C₁-C₆-Alkylgruppen mit 3 bis 8 Kohlenstoffatomen im Heteroarylrest und wenigstens einem Heteroatom ausgewählt aus N, O und S, der mit wenigstens einer Gruppe ausgewählt aus C₁-C₆-Alkylgruppen und/oder Halogenatomen substituiert sein können;
(iv) Aryl-, Aryl-C₁-C₆-Alkylgruppen mit 5 bis 12 Kohlenstoffatomen im Arylrest, die gegebenenfalls mit wenigstens einer C₁-C₆-Alkylgruppen und/oder einem Halogenatomen substituiert sein können;
und der Rest R ausgewählt ist aus
(v) linearen oder verzweigten, gesättigten oder ungesättigten, aliphatischen oder alicyclischen Alkylgruppen mit 1 bis 20 Kohlenstoffatomen;
(vi) Heteroaryl-C₁-C₆-Alkylgruppen mit 3 bis 8 Kohlenstoffatomen im Arylrest und wenigstens einem Heteroatom ausgewählt aus N, O und S, die mit wenigstens einer C₁-C₆-Alkylgruppen und/oder Halogenatomen substituiert sein können;
(vii) Aryl-C₁-C₆-Alkylgruppen mit 5 bis 12 Kohlenstoffatomen im Arylrest, die gegebenenfalls mit wenigstens einer C₁-C₆-Alkylgruppe und/oder einem Halogenenatomen substituiert sein können,
durch Alkylierung der zugrundeliegenden Amine, Phosphine, Imidazole, Pyridine, Triazole und Pyrazole mit einem Halogenid RX und Austausch des Halogenidanions X⁻ durch das oben definierte Anion [Y]⁻ oder [Y]²⁻, dadurch gekennzeichnet, dass das Verfahren ohne Isolierung der Zwischenprodukte durchgeführt wird.

Weitere bevorzugte Ausgestaltungen ergeben sich aus den Patentansprüchen.

Das erfindungsgemäße Verfahren weist die oben genannten Nachteile nicht auf. Insbesondere ist durch die erfindungsgemäße einstufige Verfahrensweise ohne Isolierung und/oder Reinigung der Zwischenprodukte ein problemloses scale-up möglich.

In einer besonderen Ausgestaltung der Erfindung können die Alkyl-, Aryl-, Arylalkyl- und Alkylaryl-Sulfonatgruppen durch Halogenatome, insbesondere Fluor, Chlor oder Brom substituiert sein. Besonders bevorzugt sind die fluorierten, insbesondere die perfluorierten Alkyl - und obengenannten Arylsulfonate, wie das Trifluormethansulfonat (Triflat). Als nicht halogenierte Vertreter sind die Methansulfonat-, Benzolsulfonat- und die Toluolsulfonat-Gruppe zu nennen, sowie alle weiteren im Stand der Technik bekannten Sulfonat-Austrittsgruppen.

In einer weiteren Ausgestaltung der Erfindung können die Alkyl-, Aryl-, Arylalkyl- und Alkylaryl-Carboxylatgruppen durch Halogenatome, insbesondere Fluor, Chlor oder Brom substituiert sein. Besonders bevorzugt sind die fluorierten, insbesondere die perfluorierten Alkyl - und obengenannten Arylcarboxylate, wie das Trifluormethancarboxylat (Trifluoracetat; CF₃COO⁻). Als nicht halogenierte Vertreter sind die Acetat- und Benzoat-Gruppe zu nennen, sowie alle weiteren im Stand der Technik bekannten Carboxylat-Austrittsgruppen.

Das im Alkylierungsagens RX enthaltende Halogenidanion X⁻ ist ausgewählt aus der Gruppe bestehend aus Chlorid, Bromid und Iodid. Unter dem Begriff "Alkylierung" ist die Alkylierung selbst aber auch die Arylalkylierung und die Heteroarylalkylierung mit den angegebenen Gruppen zu verstehen.

In bevorzugten Ausgestaltungen der Erfindung können die im Zusammenhang mit den Substituenten erwähnten C₁-C₆-Alkyl-Gruppen jeweils unabhängig voneinander durch C₂-C₄-Alkyl-Gruppen ersetzt werden. Ebenso können die im Zusammenhang mit den Substituenten erwähnten C₁-C₆-Alkoxy-Gruppen jeweils unabhängig voneinander durch C₂-C₄-Alkoxy-Gruppen ersetzt werden. In einer weiteren Alternative der Erfindung können die im Zusammenhang mit den Substituenten erwähnten C₅-C₁₂-Aryl-Gruppen jeweils unabhängig voneinander durch C₆-C₁₀-Aryl-Gruppen, die C₃-C₈-Heteroaryl-Gruppen jeweils unabhängig voneinander durch C₃-C₆-Heteroaryl-Gruppen ersetzt werden. Die Halogenatome, mit welchen die Alkyl-, Alkoxy- und Aryl-Gruppen substituiert sein können sind ausgewählt aus Fluor, Chlor, Brom und Iod.

Ein einer bevorzugten Ausgestaltung ist der Rest R' ein linearer oder verzweigter 1 bis 8 Kohlenstoffatome enthaltender aliphatischer oder alicyclischer Alkyl- oder ein C₆-C₁₀-Aryl-, C₆-C₁₀-Aryl-C₁-C₄-alkyl- oder C₁-C₄-Alkyl-C₆-C₁₀-Arylrest, der durch Halogenatome substituiert sein kann

Die Kationen sind ausgewählt aus Trimethylphenylammonium, Methyltrioctylammonium, Tetrabutylphosphonium, 3-Butyl-1-methyl-imidazolium, 3-Ethyl-1-methyl-imidazolium, N-Butylpyridinium, N-Ethylpyridinium, Diethylpyrazolium, 1-Ethyl-3-methylimidazolium, 1-Butyl-3-methylimidazolium, 1-Hexyl-3-methylimidazolium, 1-Octyl-3-methylimidazolium, 1-Decyl-3-methylimidazolium, 1-Butyl-4-methylpyridinium, 1-Butyl-3-methylpyridinium, 1-Butyl-2-methylpyridinium, 1-Butyl-pyridinium.

Bei dem erfindungsgemäßen one-pot-Verfahren reagieren ein Alkylierungsreagenzien RX, bevorzugt organische Halogenide mit
- Aminen der allgemeinen Formel

   NR¹R²R³,
- Phosphanen der allgemeinen Formel

   PR¹R²R³,
- Imidazolen der allgemeinen Formel 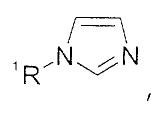 wobei der Imidazol-Kern substituiert sein kann mit Substituenten, die ausgewählt sind aus der Gruppe bestehend aus C₁-C₆-Alkyl-, C₁-C₆-Alkoxy-, C₁-C₆-Aminoalkyl-, C₅-C₁₂-Aryl- oder C₅-C₁₂-Aryl-C₁-C₆-Alkylgruppen,
- Pyridinen der allgemeinen Formel 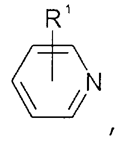 wobei der Pyridin-Kern substituiert sein kann mit Substituenten, die ausgewählt sind aus der Gruppe bestehend aus C₁-C₆-Alkyl-, C₁-C₆-Alkoxy-, C₁-C₆-Aminoalkyl-, C₅-C₁₂-Aryl- oder C₅-C₁₂-Aryl-C₁-C₆-Alkylgruppen,
- Pyrazolen der allgemeinen Formel 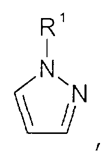 wobei der Pyrazol-Kern substituiert sein kann mit Substituenten, die ausgewählt sind aus der Gruppe bestehend aus C₁-C₆-Alkyl-, C₁-C₆-Alkoxy-, C₁-C₆-Aminoalkyl-, C₅-C₁₂-Aryl oder C₅-C₁₂-Aryl-C₁-C₆-Alkylgruppen,
- oder Triazolen der allgemeinen Formel 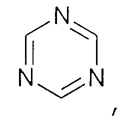 wobei der Triazol-Kern substituiert sein kann mit Substituenten, die ausgewählt sind aus der Gruppe bestehend aus C₁-C₆-Alkyl-, C₁-C₆-Alkoxy-, C₁-C₆-Aminoalkyl-, C₅-C₁₂-Aryl- oder C₅-C₁₂-Aryl-C₁-C₆-Alkylgruppen,
in Anwesenheit eines Metallsalzes der allgemeinen Formel M^{m+}[Y]⁻ₘ, M₂⁺[Y]²⁻ oder M²⁺[Y]²⁻ direkt zur gewünschten binären ionischen Flüssigkeit [A]ₙ⁺ [Y]ⁿ⁻. Bei dem Metallsalz der Formel M^{m+}[Y]⁻ₘ mit m = 1, 2 oder 3 handelt es sich bevorzugt um Alkali- oder Erdalkalimetall-, Blei- oder Silbersalze. Bei den Metallsalzen M₂⁺[Y]²⁻ oder M²⁺[Y]²⁻ handelt es sich vorzugsweise um Sulfate oder Carbonate der Alkalimetalle oder Kupfer, Nickel, Cobalt, Zink, Magnesium und Eisen.

Besonders bevorzugt lassen sich nach dem erfindungsgemäßen Verfahren ionische Flüssigkeiten Herstellen, die ausgewählt sind aus 1-Ethyl-3-methylimidazoliumtetrafluoroborat, 1-Ethyl-3-methylimidazoliumhexafluorophosphat, 1-Ethyl-3-methylimidazoliumbenzolsulfonat, 1-Butyl-3-methylimidazoliumtetrafluoroborat, 1-Butyl-3-methylimidazoliumhexafluorophosphat, 1-Butyl-3-methylimidazoliumbenzolsulfonat, 1-Hexyl-3-methylimidazoliumtetrafluoroborat, 1-Hexyl-3-methylimidazoliumhexafluorophosphat, 1-Hexyl-3-methylimidazolium benzolsulfonat, 1-Octyl-3-methylimidazoliumtetrafluoroborat, 1-Octyl-3-methylimidazoliumhexafluorophosphat, 1-Octyl-3-methylimidazoliumbenzolsulfonat, 1-Decyl-3-methylimidazoliumtetrafluoroborat, 1-Decyl-3-methylimidazoliumhexafluorophosphat, 1-Decyl-3-methylimidazolium benzolsulfonat, 1-Butyl-4-methylpyridiniumtetrafluoroborat, 1-Butyl-4-methylpyridiniumhexafluorophosphat, 1-Butyl-4-methylpyridiniumbenzolsulfonat, 1-Butyl-3-methylpyridiniumtetrafluoroborat, 1-Butyl-3-methylpyridiniumhexafluorophosphat, 1-Butyl-3-methylpyridiniumbenzolsulfonat, 1-Butyl-2-methylpyridiniumtetrafluoroborat, 1-Butyl-2-methylpyridiniumhexafluorophosphat, 1-Butyl-2-methylpyridiniumbenzolsulfonat, 1-Butyl-pyridinium tetrafluoroborat, 1-Butyl-pyridiniumhexafluorophosphat, 1-Butyl-pyridiniumbenzolsulfonat.

Das zum Anionenaustausch verwendete Metallsalz der Formel M^{m+}[Y]⁻ₘ, M₂⁺[Y]²⁻ oder M²⁺[Y]²⁻kann bereits zu Reaktionsbeginn in die Reaktion eingesetzt werden oder zu jedem späteren Zeitpunkt der Reaktionsmischung zugegeben werden.

Die erfindungsgemäße one-pot Reaktion kann bei Temperaturen von -10 bis 250 °C, bevorzugt zwischen 30 bis 130 °C, besonders bevorzugt zwischen 60 und 100°C durchgeführt werden.

Die verfahrensgemäßen Reaktionszeiten liegen zwischen 5 min und 300h, bevorzugt zwischen 2h und 120h, besonders bevorzugt zwischen 2h und 48h.

Während der Reaktion werden die Reaktionskomponenten miteinander vermischt.

Die Reinigung der ionischen Flüssigkeiten erfolgt durch einfaches Abtrennen der ausgefällten Metallhalogenide aus dem Metallkation M und dem Anion X, durch gängige, im Stand der Technik beschriebene Verfahren zur Abtrennung von Feststoffen und anschließendem Entfernen des gegebenenfalls vorhandenen Lösungsmittels und/oder des überschüssigen Alkylierungsreagenzes RX. Die dazu geeigneten Verfahren sind dem Fachmann aus dem Stand der Technik bekannt.

Durch diese Verfahrensweise entfällt der Aufarbeitungsschritt unter Handhabung des extrem hygroskopischen Halogenidsalzes. Zusätzlich hilft das erfindungsgemäße Verfahren, die Produktreinheit wesentlich zu verbessern. Da das hygroskopische Halogenidsalz im Verfahren gemäß dieser Erfindung nicht mehr isoliert und gehandhabt wird, kann der damit zwangsläufig verbundene Eintrag von Wasserspuren vollständig vermieden werden. Da der Wassergehalt von entscheidender Bedeutung für die Menge an Halogenidverunreinigungen im Produkt ist, ergibt sich auf diese Weise durch das neue Verfahren ein deutlicher Vorteil in der Produktqualität.

So konnte beim Versetzen der, durch ein erfindungsgemäßes Verfahren hergestellten, ionischen Flüssigkeiten mit Silbernitratlösung keine Bildung von Silberchloridniederschlägen beobachtet werden. Bei den gleichen ionischen Flüssigkeiten, die jedoch nach einem Verfahren des Standes der Technik hergestellt wurden, signalisierte der beim Versetzen mit Silbernitratlösung entstehende Silberchloridniederschlag die Anwesenheit von Halogenid-Verunreinigungen.

Ein weiterer wirtschaftlicher Vorteil ergibt sich für das erfindungsgemäße Verfahren aus der Tatsache, dass kein weiteres, hochreines und trockenes Lösungsmittel für den Anionaustausch benötigt wird. Das Trocknen und Absolutieren des Lösungsmittels für die Austauschreaktion ist bekanntermaßen ein langwieriger und kostenintensiver Prozess. Stattdessen kann das erfindungsgemäße Eintopf-Verfahren auch in einem Überschuss an Alkylierungsmittel RX als Lösungsmittel durchgeführt werden.

Das molare Verhältnis des eingesetzten Alkylierungsmittels RX kann, bezogen auf die zugrundeliegenden Amine, Phosphine, Imidazole, Pyrazole, Triazole oder Pyridine, 1 bis 20, bevorzugt 1 bis 5 , besonders bevorzugt 1 bis 3 betragen.

Das überschüssige Alkylierungsmittel RX kann bei der Produktisolierung destillativ zurückgewonnen und wiederverwendet werden.

Zudem können bei dem erfindungsgemäßen einstufigen Verfahren zusätzliche organische Lösungsmittel in die one-pot Alkylierungs-/ Austauschreaktion eingesetzt werden. Bevorzugte organische Lösungsmittel sind solche die einen Siedepunkt unterhalb 150 °C besitzen, besonders bevorzugt sind Chlorbenzol, Methylenchlorid, Aceton, Acetonitril, Essigester, Methanol, Ethanol, Toluol oder Gemische dieser organischen Lösungsmittel. Bevorzugt sind Lösungsmittel mit einem Siedepunkt von weniger als 120°C. Dabei kann das molare Verhältnis des zusätzlich eingesetzten Lösungsmittels, bezogen auf die zugrundeliegenden Amine, Phosphine, Imidazole, Pyrazole, Triazole oder Pyridine, 0,05 bis 20, bevorzugt 0,5 bis 5, besonders bevorzugt 0,5 bis 2 betragen.

Das zusätzlich eingesetzte Lösungsmittel kann bei der Produktisolierung destillativ zurückgewonnen und wiederverwendet werden.

Die Erfindung wird durch die nachfolgenden Beispiele näher beschrieben.

### Beispiele

### 1. Synthese der ionische Flüssigkeit 1-Butyl-3-methy-imidazoliumtetrafluoroborat nach Stand der Technik (Referenzbeispiel)

Nach einer Vorschrift von Wilkes (J. S. Wilkes, J. A. Levisky, R. A. Wilson, C. L. Hussey, *Inorg*. *Chem.,* **1982,** 21, 1263) wird in einem ersten Schritt das 1-Butyl-3-methyl-imidazolium chloride wie folgt hergestellt. In einen 500 ml Schlenkkolben mit Rückflusskühler werden 46,0 g (0,56 mol) 1-Methylimidazol und 77,8 g (0,84 mol) Butylchlorid zusammengegeben und bei 80°C ca. 3 Tage gerührt. Anschließend wir das überschüssige Butylchlorid abdekantiert und das Produkt bei 80°C im HV getrocknet. Man erhält 80,2 g des weiß, gelblichen, extrem hygroskopischen Feststoffs, 1-Butyl-3-methyl-imidazolium chlorid. 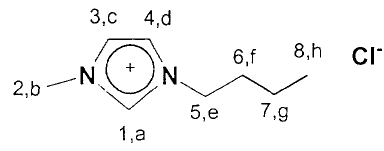 ^{**1**}**H-NMR (300 MHz, CDCl**_{**3**}**):** δ=9.9 ppm (s,1H,Ha); δ=7.26 ppm (d, 1H, H_{c}); δ=7.24 ppm (d,1H,H_{d}); δ=4.1 ppm (t,2H,Hₑ); δ=3.9 ppm (s,2H,H_{b}); δ=1.6 ppm (m,2H,H_{f}); δ=1.2 ppm (m,2H,H_{g}); δ=0.8 ppm (t,3H,Hₕ).
^{**13**}**C-NMR (75 MHz, CDCl**_{**3**}**):** δ=137 ppm (C1); δ=124-122 ppm (C3,C4); δ=50 ppm (C5); δ=36 ppm (C2); δ=33 ppm (C6); δ=20 ppm (C7); δ=14 ppm (C8).

In einem zweiten Schritt wird nach einer Vorschrift Fuller (P.A.Z. Suarez, J.E.L. Dullius, S. Einloft, R.F. de Souza, J. Dupont, *Polyhedron,* **1996,** *15,* 1217-1219) aus dem 1-Butyl-3-methyl-imidazolium chloride durch Anionenaustausch das 1-Butyl-3-methyl-imidazoliumtetrafluoroborat hergestellt.

Die im ersten Schritt hergestellten 80,2 g (0,46 mol) 1-Butyl-3-methylimidazolium chloride werden in ca. 200 ml absolutem Aceton gelöst und mit 75,62 g (0,69 mol) Natriumtetrafluoroborat versetzt und für ca. 1 Woche bei 70°C gerührt. Der entstandene Feststoff wird über eine Schutzgasfritte abfiltriert und das Filtrat am Rotationsverdampfer vom Lösungsmittel befreit und über Nacht bei 60°C im HV getrocknet. Man erhält 1-Butyl-3-methyl-imidazoliumtetrafluoroborat in 74 %iger Ausbeute. Zum qualitativen Nachweis auf Chloridreste, werden ca. 1 ml des Produktes mit ca. 5 ml Wasser versetzt und mit 2 Tropfen konzentrierter Salpetersäure angesäuert. Zu dieser Lösung werden dann ca. 3-4 Tropfen Silbernitrat gegeben um etwaig vorhandenes Chlorid als Silberchlorid auszufällen. Ein weißer Niederschlag spricht für eine nicht vollständige Chloridfreiheit des Produktes. 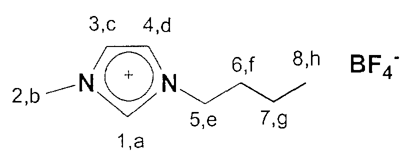 ^{**1**}**H-NMR (300 MHz, CDCl**_{**3**}**):** 0,79 (3 H, tr, J=5,8 Hz, Hₐ); 1,22 (2 H, m, H_{b}); 1,74 (2 H, m, H_{c}); 3,86 (3 H, s, Hₕ); 4,17 (2 H, tr, J=5,8 Hz, H_{d}); 7,51; 7,57 (je 1 H, s, H_{f,g}); 8,7 (1 H, s, Hₑ).
^{**13**}**C-NMR (75 MHz, CDCl**_{**3**}**):** 14,1 (a); 20,3-36,8 (b, c, d); 50,4 (h); 123,2-125,0 (f,g); 137,8 (e).
^{**19**}**F-NMR (281 MHz, CDCl**_{**3**}**):** 148,5-150,8 (m)

### 2. Synthese der ionische Flüssigkeit 1-Ethyl-3-methy-imidazoliumtetrafluoroborat

In einen 1 I Schlenkkolben werden 114,5 g (1,39 mol) 1-Methylimidazol, 304,0 g (2,79 mol) Ethylbromid und 228,9 g (2,09 mol) Natriumtetrafluorborat gegeben und bei 70°C ca. 2 Wochen gerührt. Der entstandene weiße Feststoff wird über eine Schutzgasfritte abfiltriert und die sich gebildeten zwei Phasen voneinander mit Hilfe eines Scheidetrichters getrennt. Die untere gelbliche Phase wird über Nacht bei 60°C im HV getrocknet. Man erhält 1-Ethyl-3-methylimidazoliumtetrafluoroborat in 87 %iger Ausbeute. Zum qualitativen Nachweis auf Chloridreste, werden ca. 1 ml des Produktes mit ca. 5 ml Wasser versetzt und mit 2 Tropfen konzentrierter Salpetersäure angesäuert. Zu dieser Lösung werden dann ca. 3-4 Tropfen Silbernitrat gegeben um etwaig vorhandenes Chlorid als Silberchlorid auszufällen. Das Ausbleiben eines Niederschlags spricht für die komplette Abwesenheit von Chloridresten. 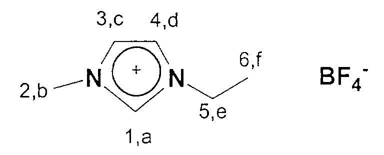 ^{**1**}**H-NMR (300 MHz, CDCl**_{**3**}**):** δ=9.6 ppm (s,1H,Hₐ); δ=7.26 ppm (d,1H,H_{c}); δ=7.24 ppm (d,1H,H_{d}); δ=3.9 ppm (q,2H,Hₑ); δ=3,6 ppm (s,3H,H_{b}); δ=1.2 ppm (t,3H,H_{f}).
^{**13**}**C-NMR (75 MHz, CDCl**_{**3**}**):** δ=137 ppm (C1); δ=124-122 ppm (C3,C4); δ=45 ppm (C5); δ=36 ppm (C2); δ=16 ppm (C6).
^{**19**}**F-NMR (281 MHz, CDCl**_{**3**}**):** δ= -151,3 ppm (d, ¹J(BF)= 22 Hz)

### 3. Synthese der ionische Flüssigkeit 1-Ethyl-3-methyimidazoliumhexafluorophosphat

In einen 1 I Schlenkkolben werden 92,1 g (1,20 mol) 1-Methylimidazol, 261,5 (2,40 mol) Ethylbromid und 302,3 g (1,80 mol) Natriumhexafluorophosphat gegeben und bei 70°C ca. 2 Wochen gerührt. Der entstandene weiße Feststoff wird über eine Schutzgasfritte abfiltriert. Der Filterkuchen wird zwei mal mit je 400 ml Methylenchlorid gewaschen, die Phasen vereinigt und von dem Methylenchlorid befreit. Danach wird der weiße Feststoff über Nacht bei 60°C im HV getrocknet. Man erhält 1-Ethyl-3-methyl-imidazolium hexafluorophosphat in 92 %iger Ausbeute. Zum qualitativen Nachweis auf Chloridreste, werden ca. 1 g des Produktes in ca. 5 ml Wasser gelöst und mit 2 Tropfen konzentrierter Salpetersäure angesäuert. Zu dieser Lösung werden dann ca. 3-4 Tropfen Silbernitrat gegeben um etwaig vorhandenes Chlorid als Silberchlorid auszufällen. Das Ausbleiben eines Niederschlags spricht für die komplette Abwesenheit von Chloridresten. 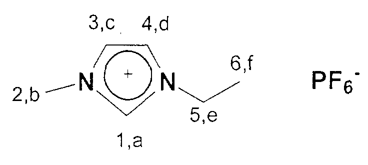 ^{**1**}**H-NMR (300 MHz, CDCl**_{**3**}**):** δ=9.6 ppm (s,1H,Hₐ); δ=7.26 ppm (d,1H,H_{c}); δ=7.24 ppm (d,1H,H_{d}); δ=3.9 ppm (q,2H,Hₑ); δ=3.6 ppm (s,3H,H_{b}); δ=1.2 ppm (t,3H,H_{f}).
^{**13**}**C-NMR (75 MHz, CDCl**_{**3**}**):** δ=137 ppm (C1); δ=124-122 ppm (C3,C4); δ=45 ppm (C5); δ=36 ppm (C2); δ=16 ppm (C6).
^{**31**}**P-NMR (121 Mhz):** -143.08 (Heptett, J=710 Hz).
^{**19**}**F-NMR (281 Mhz): -** 72.5 (d, J=710 Hz).

### 4. Synthese der ionische Flüssigkeit 1-Butyl-3-methyimidazoliumtetrafluoroborat

In einen 1 I Schlenkkolben werden 114,5 g (1,39 mol) 1-Methylimidazol, 258,1 g (2,79 mol) Butylchlorid und 228,9 g (2,09 mol) Natriumtetrafluoroborat gegeben und bei 70°C ca. 2 Wochen gerührt. Der entstandene weiße Feststoff wird über eine Schutzgasfritte abfiltriert und die sich gebildeten zwei Phasen voneinander mit Hilfe eines Scheidetrichters getrennt. Die untere gelbliche Phase wird über Nacht bei 60°C im HV getrocknet. Man erhält 1-Butyl-3-methylimidazolium tetrafluoroborat in 90 %iger Ausbeute. Zum qualitativen Nachweis auf Chloridreste, werden ca. 1 ml des Produktes mit ca. 5 ml Wasser versetzt und mit 2 Tropfen konzentrierter Salpetersäure angesäuert. Zu dieser Lösung werden dann ca. 3-4 Tropfen Silbernitrat gegeben um etwaig vorhandenes Chlorid als Silberchlorid auszufällen. Das Ausbleiben eines Niederschlags spricht für die komplette Abwesenheit von Chloridresten. 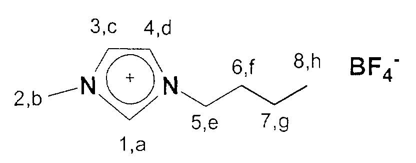 ^{**1**}**H-NMR (300 MHz, CDCl**_{**3**}**):** 0,79 (3 H, tr, J=5,8 Hz, Hₐ); 1,22 (2 H, m, H_{b}); 1,74 (2 H, m, H_{c}); 3,86 (3 H, s, Hₕ); 4,17 (2 H, tr, J=5,8 Hz, H_{d}); 7,51; 7,57 (je 1 H, s, H_{f,g}); 8,7 (1 H, s, Hₑ).
^{**13**}**C-NMR (75 MHz, CDCl**_{**3**}**):** 14,1 (a); 20,3-36,8 (b, c, d); 50,4 (h); 123,2-125,0 (f,g); 137,8 (e).
^{**19**}**F-NMR (281 MHz, CDCl**_{**3**}**):** 148,5-150,8 (m)

### 5. Synthese der ionische Flüssigkeit 1-Butyl-3-methyimidazoliumhexafluorophosphat

In einen 1 I Schlenkkolben werden 92,1 g (1,20 mol) 1-Methylimidazol, 222,2 g (2,40 mol) Butylchlorid und 302,3 g (1,80 mol) Natriumhexafluorophosphat gegeben und bei 70°C ca. 2 Wochen gerührt. Der entstandene weiße Feststoff wird über eine Schutzgasfritte abfiltriert und die sich gebildeten zwei Phasen voneinander mit Hilfe eines Scheidetrichters getrennt. Die untere gelbliche Phase wird über Nacht bei 60°C im HV getrocknet. Man erhält 1-Butyl-3-methylimidazoliumhexafluorophosphat in 92 %iger Ausbeute. Zum qualitativen Nachweis auf Chloridreste, werden ca. 1 ml des Produktes mit ca. 5 ml Wasser versetzt und mit 2 Tropfen konzentrierter Salpetersäure angesäuert. Zu dieser Lösung werden dann ca. 3-4 Tropfen Silbernitrat gegeben um etwaig vorhandenes Chlorid als Silberchlorid auszufällen. Das Ausbleiben eines Niederschlags spricht für die komplette Abwesenheit von Chloridresten. 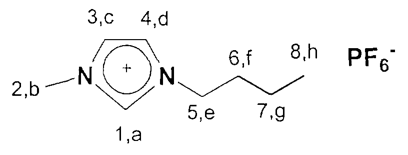 ^{**1**}**H-NMR (300 MHz, CDCl**_{**3**}**):** 0,77 (3 H, tr, J=5,8 Hz, Hₐ); 1,05-1,17 (2 H, m, H_{b}); 1,50-1,59 (2 H, m, H_{c}); 3,51 (3 H, s, Hₕ); 3,75 (2 H, tr, J=5,8 Hz, H_{d}); 6,95-7,26 (je 1 H, s, H_{f,g}); 7,97 (1 H, s, Hₑ).
^{**13**}**C-NMR (75 MHz, CDCl**_{**3**}**):** 13,3 (a); 19,4-35,7 (b, c, d); 49,5 (h); 122,2-123,5 (f,g); 135,7 (e).
^{**31**}**P-NMR (121 MHz, CDCl**_{**3**}**):** -143,08 (Heptett, J=710Hz)
^{**19**}**F-NMR (281 MHz, CDCl**_{**3**}**):** -72,5 (d, J=710 Hz)

### 6. Synthese der ionische Flüssigkeit 1-Hexyl-3-methy-imidazoliumtetrafluoroborat

In einen 1 I Schlenkkolben werden 126,0 g (1,53 mol) 1-Methylimidazol, 370,2 g (3,07 mol) Hexylchlorid und 251,8 g (2,30 mol) Natriumtetrafluoroborat gegeben und bei 70°C ca. 2 Wochen gerührt. Der entstandene weiße Feststoff wird über eine Schutzgasfritte abfiltriert und die sich gebildeten zwei Phasen voneinander mit Hilfe eines Scheidetrichters getrennt. Die untere gelbliche Phase wird über Nacht bei 60°C im HV getrocknet. Man erhält 1-Hexyl-3-methylimidazoliumtetrafluoroborat in 91 %iger Ausbeute. Zum qualitativen Nachweis auf Chloridreste, werden ca. 1 ml des Produktes mit ca. 5 ml Wasser versetzt und mit 2 Tropfen konzentrierter Salpetersäure angesäuert. Zu dieser Lösung werden dann ca. 3-4 Tropfen Silbernitrat gegeben um etwaig vorhandenes Chlorid als Silberchlorid auszufällen. Das Ausbleiben eines Niederschlags spricht für die komplette Abwesenheit von Chloridresten. 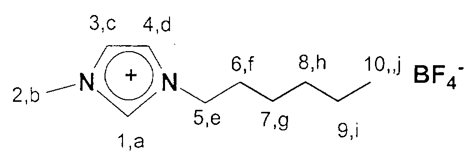 ^{**1**}**H-NMR (300 MHz, CDCl**_{**3**}**):** δ=9.9 ppm (s,1H,Hₐ); δ=7.26 ppm (d,1H,H_{c}); δ=7.24 ppm (d,1H,H_{d}); δ=4.1 ppm (t,2H,Hₑ); δ=3.9 ppm (s,2H,H_{b}); δ=1.6 ppm (m,2H,H_{f}); δ=1.2 ppm (m,6H,H_{g,h,i}); δ=0.8 ppm (t,3H,H_{J}).
^{**13**}**C-NMR (75 MHz, CDCl**_{**3**}**):** δ=137 ppm (C1); δ=124-122 ppm (C3,C4); δ=48 ppm (C5); δ=35 ppm (C2); δ=29-24 ppm (C6-C8); δ=20 ppm (C9); δ=13 ppm (C10).
^{**19**}**F-NMR (281 MHz, CDCl**_{**3**}**):** δ= -151,3 ppm (d, ¹J(BF)= 22 Hz)

### 7. Synthese der ionische Flüssigkeit 1-Hexyl-3-methyl-imidazoliumhexafluorophosphat

In einen 1 I Schlenkkolben werden 82,9 g (1,08 mol) 1-Methylimidazol, 260,5 g (2,16 mol) Hexylchlorid und 272,1 g (1,62 mol) Natriumhexafluorophosphat gegeben und bei 70°C ca. 2 Wochen gerührt. Der entstandene weiße Feststoff wird über eine Schutzgasfritte abfiltriert und die sich gebildeten zwei Phasen voneinander mit Hilfe eines Scheidetrichters getrennt. Die untere gelbliche Phase wird über Nacht bei 60°C im HV getrocknet. Man erhält 1-Hexyl-3-methyl-imidazolium hexafluorophosphat in 87 %iger Ausbeute. Zum qualitativen Nachweis auf Chloridreste, werden ca. 1 ml des Produktes mit ca. 5 ml Wasser versetzt und mit 2 Tropfen konzentrierter Salpetersäure angesäuert. Zu dieser Lösung werden dann ca. 3-4 Tropfen Silbernitrat gegeben um etwaig vorhandenes Chlorid als Silberchlorid auszufällen. Das Ausbleiben eines Niederschlags spricht für die komplette Abwesenheit von Chloridresten. 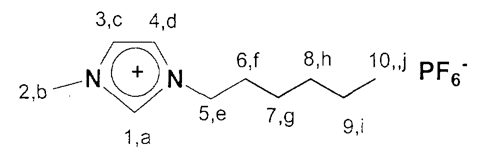 ^{**1**}**H-NMR (300 MHz, CDCl**_{**3**}**):** δ=9.9 ppm (s,1H,Hₐ); δ=7.26 ppm (d,1H,H_{c}); δ=7.24 ppm (d,1H,H_{d}); δ=4.1 ppm (t,2H,Hₑ); δ=3.9 ppm (s,2H,H_{b}); δ=1.6 ppm (m,2H,H_{f}); δ=1.2 ppm (m,6H,H_{g,h,i}); δ=0.8 ppm (t,3H,Hⱼ).
^{**13**}**C-NMR (75 MHz, CDCl**_{**3**}**):** δ=137 ppm (C1); δ=124-122 ppm (C3,C4); δ=48 ppm (C5); δ=35 ppm (C2); δ=29-24 ppm (C6-C8); δ=20 ppm (C9); δ=13 ppm (C10).
^{**31**}**P-NMR (121 Mhz):** -143.08 (Heptett, J=710 Hz).
^{**19**}**F-NMR (281 Mhz): -** 72.5 (d, J=710 Hz).

### 8. Synthese der ionischen Flüssigkeit 1-Octyl-3-methy-imidazoliumtetrafluoroborat

In einen 1 I Schlenkkolben werden 120,2 g (1,46 mol) 1-Methylimidazol, 435,6 g (2,93 mol) Octylchlorid und 240,3 g (2,19 mol) Natriumtetrafluoroborat gegeben und bei 70°C ca. 2 Wochen gerührt. Der entstandene weiße Feststoff wird über eine Schutzgasfritte abfiltriert und die sich gebildeten zwei Phasen voneinander mit Hilfe eines Scheidetrichters getrennt. Die untere gelbliche Phase wird über Nacht bei 60°C im HV getrocknet. Man erhält 1-Octyl-3-methylimidazoliumtetrafluoroborat in 90 %iger Ausbeute. Zum qualitativen Nachweis auf Chloridreste, werden ca. 1 ml des Produktes mit ca. 5 ml Wasser versetzt und mit 2 Tropfen konzentrierter Salpetersäure angesäuert. Zu dieser Lösung werden dann ca. 3-4 Tropfen Silbernitrat gegeben um etwaig vorhandenes Chlorid als Silberchlorid auszufällen. Das Ausbleiben eines Niederschlags spricht für die komplette Abwesenheit von Chloridresten. 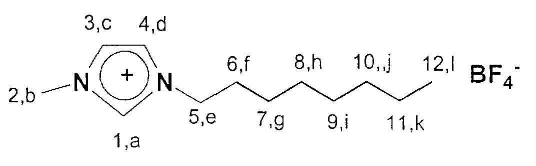 ^{**1**}**H-NMR (300 MHz, CDCl**_{**3**}**):** δ=9.3 ppm (s,1H,Hₐ); δ=7.26 ppm (d,1H,H_{c}); δ=7.24 ppm (d,1H,H_{d}); δ=4.1 ppm (t,2H,Hₑ); δ=3.9 ppm (s,2H,H_{b}); δ=1.6 ppm (m,2H,H_{f}); δ=1.2 ppm (m,10H,H_{g,h,i,j,k}); δ=0.8 ppm (t,3H,Hₗ).
^{**13**}**C-NMR (75 MHz, CDCl**_{**3**}**):** δ=137 ppm (C1); δ=124-122 ppm (C3,C4); δ=48 ppm (C5); δ=35 ppm (C2); δ=29-24 ppm (C6-C10); δ=20 ppm (C11); δ=13 ppm (C12).
^{**19**}**F-NMR (281 MHz, CDCl**_{**3**}**):** δ= -151,3 ppm (d, ¹J(BF)= 22 Hz)

### 9. Synthese der ionische Flüssigkeit 1-Octyl-3-methy-imidazoliumhexafluorophosphat

In einen 1 I Schlenkkolben werden 92,1 g (1,20 mol) 1-Methylimidazol, 356,8 g (2,40 mol) Octylchlorid und 302,3 g (1,80 mol) Natriumhexafluorophosphat gegeben und bei 70°C ca. 2 Wochen gerührt. Der entstandene weiße Feststoff wird über eine Schutzgasfritte abfiltriert und die sich gebildeten zwei Phasen voneinander mit Hilfe eines Scheidetrichters getrennt. Die untere gelbliche Phase wird über Nacht bei 60°C im HV getrocknet. Man erhält 1-Octyl-3-methyl-imidazolium hexafluorophosphat in 82 %iger Ausbeute. Zum qualitativen Nachweis auf Chloridreste, werden ca. 1 ml des Produktes mit ca. 5 ml Wasser versetzt und mit 2 Tropfen konzentrierter Salpetersäure angesäuert. Zu dieser Lösung werden dann ca. 3-4 Tropfen Silbernitrat gegeben um etwaig vorhandenes Chlorid als Silberchlorid auszufällen. Das Ausbleiben eines Niederschlags spricht für die komplette Abwesenheit von Chloridresten. 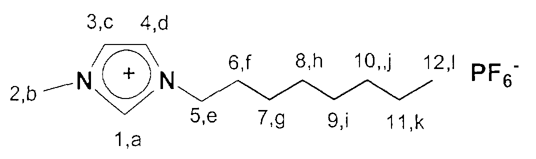 ^{**1**}**H-NMR (300 MHz, CDCl**_{**3**}**):** δ=9.3 ppm (s,1H,Hₐ); δ=7.26 ppm (d,1H,H_{c}); δ=7.24 ppm (d,1H,H_{d}); δ=4.1 ppm (t,2H,Hₑ); δ=3.9 ppm (s,2H,H_{b}); δ=1.6 ppm (m,2H,H_{f}); δ=1.2 ppm (m,10H,H_{g,h,i,j,k}); δ=0.8 ppm (t,3H,Hₗ).
^{**13**}**C-NMR (75 MHz, CDCl**_{**3**}**):** δ=137 ppm (C1); δ=124-122 ppm (C3,C4); δ=48 ppm (C5); δ=35 ppm (C2); δ=29-24 ppm (C6-C10); δ=20 ppm (C11); δ=13 ppm (C12).
^{**31**}**P-NMR (121 Mhz):** -143.08 (Heptett, J=710 Hz).
^{**19**}**F-NMR (281 Mhz): -** 72.5 (d, J=710 Hz).

### 10. Synthese der ionische Flüssigkeit 1-Ethyl-3-methy-imidazoliumbenzolsulfonat

In einen 1 I Schlenkkolben werden 92,1 g (1,20 mol) 1-Methylimidazol, 261,5 g (2,40 mol) Ethylbromid und 324,3 g (1,80 mol) Natriumbenzolsulfonat gegeben und bei 70°C ca. 2 Wochen gerührt. Der entstandene weiße Feststoff wird über eine Schutzgasfritte abfiltriert. Der Filterkuchen wird zwei mal mit je 400 ml Methylenchlorid gewaschen, die Phasen vereinigt und von dem Methylenchlorid befreit. Danach wird der weiße Feststoff über Nacht bei 60°C im HV getrocknet. Man erhält 1-Ethyl-3-methylimidazolium benzolsulfonat in 75 %iger Ausbeute. Zum qualitativen Nachweis auf Chloridreste, werden ca. 1 ml des Produktes mit ca. 5 ml Wasser versetzt und mit 2 Tropfen konzentrierter Salpetersäure angesäuert. Zu dieser Lösung werden dann ca. 3-4 Tropfen Silbernitrat gegeben um etwaig vorhandenes Chlorid als Silberchlorid auszufällen. Das Ausbleiben eines Niederschlags spricht für die komplette Abwesenheit von Chloridresten. 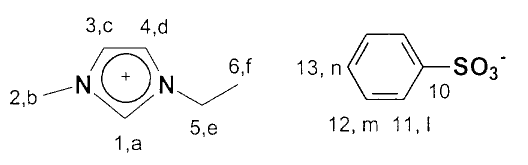 ^{**1**}**H-NMR (300 MHz, CDCl**_{**3**}**):** δ=9.3 ppm (s,1H,Hₐ); δ=7.7 ppm (d,2H,Hₗ); δ=7.26 ppm (d,H,H_{c}); δ=7.24 ppm (d,1H,H_{d}); δ=7.1 ppm (m,3H,H_{m,n}); δ=3.9 ppm (q,2H,Hₑ); δ=3.6 ppm (s,3H,H_{b}); δ=1.2 ppm (t,3H,H_{f}).
^{**13**}**C-NMR (75 MHz, CDCl**_{**3**}**):** δ=147 ppm (C10); δ=137 ppm (C1); δ=129-126 ppm (C11-13); δ=124-122 ppm (C3,C4); δ=45 ppm (C5); δ=36 ppm (C2); δ=16 ppm (C6).

### 11. Synthese der ionische Flüssigkeit 1-Butyl-3-methy-imidazoliumbenzolsulfonat

In einen 1 I Schlenkkolben werden 92,1 g (1,20 mol) 1-Methylimidazol, 222,2 g (2,40 mol) Butylchlorid und 324,3 g (1,80 mol) Natriumbenzolsulfonat gegeben und bei 70°C ca. 2 Wochen gerührt. Der entstandene weiße Feststoff wird über eine Schutzgasfritte abfiltriert und die sich gebildeten zwei Phasen voneinander mit Hilfe eines Scheidetrichters getrennt. Die untere gelbliche Phase wird über Nacht bei 60°C im HV getrocknet. Man erhält 1-Butyl-3-methylimidazolium benzolsulfonat in 78 %iger Ausbeute. Zum qualitativen Nachweis auf Chloridreste, werden ca. 1 ml des Produktes mit ca. 5 ml Wasser versetzt und mit 2 Tropfen konzentrierter Salpetersäure angesäuert. Zu dieser Lösung werden dann ca. 3-4 Tropfen Silbernitrat gegeben um etwaig vorhandenes Chlorid als Silberchlorid auszufällen. Das Ausbleiben eines Niederschlags spricht für die komplette Abwesenheit von Chloridresten. 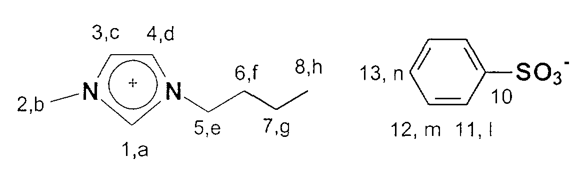 ^{**1**}**H-NMR (300 MHz, CDCl**_{**3**}**):** δ=9.6 ppm (s,1H,Hₐ); δ=7.7 ppm (d,2H,Hₗ); δ=7.26 ppm (d,1H,H_{c}); δ=7.24 ppm (d,1H,H_{d}); δ=7.1 ppm (m,3H,H_{m,n}); δ=3.9 ppm (q,2H,Hₑ); δ=3.6 ppm (s,3H,H_{b}); δ=1.2 ppm (t,3H,H_{f}).
^{**13**}**C-NMR (75 MHz, CDCl**_{**3**}**):** δ=147 ppm (C10); δ=137 ppm (C1); δ=129-126 ppm (C11-13); δ=124-122 ppm (C3,C4); δ=45 ppm (C5); δ=36 ppm (C2); δ=16 ppm (C6).

### 12. Synthese der ionische Flüssigkeit 1-Butyl-3-methy-imidazoliumbenzolsulfonat

In einen 1 I Schlenkkolben werden 87,5 g (1,14 mol) 1-Methylimidazol, 275,1 g (2,28 mol) Hexylchlorid und 308,1 g (1,71 mol) Natriumbenzolsulfonat gegeben und bei 70°C ca. 2 Wochen gerührt. Der entstandene weiße Feststoff wird über eine Schutzgasfritte abfiltriert und die sich gebildeten zwei Phasen voneinander mit Hilfe eines Scheidetrichters getrennt. Die untere gelbliche Phase wird über Nacht bei 60°C im HV getrocknet. Man erhält 1-Hexyl-3-methylimidazolium benzolsulfonat in 78 %iger Ausbeute. Zum qualitativen Nachweis auf Chloridreste, werden ca. 1 ml des Produktes mit ca. 5 ml Wasser versetzt und mit 2 Tropfen konzentrierter Salpetersäure angesäuert. Zu dieser Lösung werden dann ca. 3-4 Tropfen Silbernitrat gegeben um etwaig vorhandenes Chlorid als Silberchlorid auszufällen. Das Ausbleiben eines Niederschlags spricht für die komplette Abwesenheit von Chloridresten. 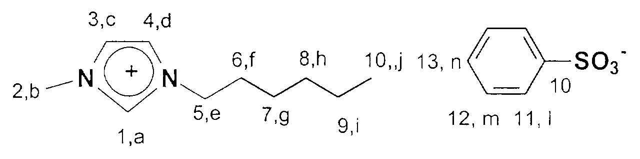 ^{**1**}**H-NMR (300 MHz, CDCl**_{**3**}**):** δ=9.9 ppm (s,1H,Hₐ); δ=7.7 ppm (d,2H,Hₗ); δ=7.26 ppm (d,1H,H_{c}); δ=7.24 ppm (d,1H,H_{d}); δ=7.1 ppm (m,3H,H_{m,n}); δ=4.1 ppm (t,2H,Hₑ); δ=3.9 ppm (s,2H,H_{b}); δ=1.6 ppm (m,2H,H_{f}); δ=1.2 ppm (m,6H,H_{g,h,i}); δ=0.8 ppm (t,3H,H_{J}).
^{**13**}**C-NMR (75 MHz, CDCl**_{**3**}**):** δ=147 ppm (C10); δ=137 ppm (C1); δ=129-126 ppm (C11-13); δ=124-122 ppm (C3,C4); δ=48 ppm (C5); δ=35 ppm (C2); δ=29-24 ppm (C6-C8); δ=20 ppm (C9); δ=13 ppm (C10).

### 13. Synthese der ionische Flüssigkeit Methyltrioctylammoniumbenzolsulfonat

In einen 1 I Autoklaven werden 282,9 g (0,80 mol) Trioctylamin und 216,2 g (1,20 mol) Natriumbenzolsulfonat gegeben. Dazu werden dann 80,8 g (1,60 mol) Methylchlorid aus einer Gaskartusche in den Autoklav geleitet und das Reaktionsgemisch bei 50°C ca. 5 Tage gerührt. Der entstandene weiße Feststoff wird über eine Schutzgasfritte abfiltriert und die sich gebildeten zwei Phasen voneinander mit Hilfe eines Scheidetrichters getrennt. Die untere gelbliche Phase wird über Nacht bei 60°C im HV getrocknet. Man erhält Methyltrioctylammonium benzolsulfonat in 63 %iger Ausbeute. Zum qualitativen Nachweis auf Chloridreste, werden ca. 1 g des Produktes in ca. 5 ml Wasser gelöst und mit 2 Tropfen konzentrierter Salpetersäure angesäuert. Zu dieser Lösung werden dann ca. 3-4 Tropfen Silbernitrat gegeben um etwaig vorhandenes Chlorid als Silberchlorid auszufällen. Das Ausbleiben eines Niederschlags spricht für die komplette Abwesenheit von Chloridresten. 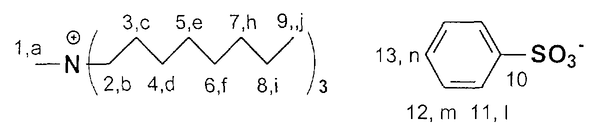 ^{**1**}**H-NMR (300 MHz, CDCl**_{**3**}**):** δ=7.4 ppm (d,2H,Hₗ); δ=7.0 ppm (d,2H,Hₘ); δ=2.8 ppm (m,6H,H_{b}), δ=2.7 ppm (s,3H,Hₐ); δ=1.2 ppm (m,6H,Hₕ); δ=1.0 ppm (m,24H,H_{c,d,e,f}); δ=0.8 ppm (t,9H,Hⱼ).
^{**13**}**C-NMR (75 MHz, CDCl**_{**3**}**):** δ=144-126 ppm (C10-C13); δ= 47 ppm (C2); δ= 34-22 ppm (C3-C7); δ= 21 ppm (C1); δ= 13 ppm (C8).

### 14. Synthese der ionische Flüssigkeit 4-Methyl-N-butylpyridiniumtetrafluoroborat

In einen 1 I Schlenkkolben werden 129,5 g (1,39 mol) 4-Picolin, 258,1 g (2,79 mol) Butylchlorid und 228,9 g (2,09 mol) Natriumtetrafluoroborat gegeben und bei 70°C ca. 2 Wochen gerührt. Der entstandene weiße Feststoff wird über eine Schutzgasfritte abfiltriert und die sich gebildeten zwei Phasen voneinander mit Hilfe eines Scheidetrichters getrennt. Die untere gelbliche Phase wird über Nacht bei 60°C im HV getrocknet. Man erhält 4-Methyl-N-butyl-pyridinium tetrafluoroborat in 84 %iger Ausbeute. Zum qualitativen Nachweis auf Chloridreste, werden ca. 1 ml des Produktes mit ca. 5 ml Wasser versetzt und mit 2 Tropfen konzentrierter Salpetersäure angesäuert. Zu dieser Lösung werden dann ca. 3-4 Tropfen Silbernitrat gegeben um etwaig vorhandenes Chlorid als Silberchlorid auszufällen. Das Ausbleiben eines Niederschlags spricht für die komplette Abwesenheit von Chloridresten. 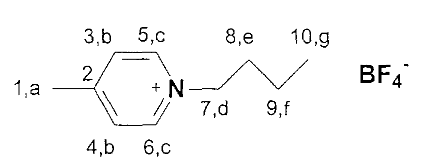 ^{**1**}**H-NMR (300 MHz, CDCl**_{**3**}**:** 0,79 (3 H, tr, J=7,45 Hz, Hₐ); 1,24 (2 H, mult, H_{b}); 1,81 (2 H, mult, H_{c}); 2,50 (3 H, s, Hₖ); 4,43 (2 H, tr, J=7,2 Hz, H_{d;}); 7,72 ( 2 H, H_{g,h}); 8,56 (2 H, H_{e,f}).
^{**13**}**C-NMR (75 Mhz, CDCl**_{**3**}**:** 13,1 (a); 18,9 (b); 21,6 (k); 33,0 (c); 60,8 (d); 128,8 (g,h); 143,3 (i); 159,3 (e,f).
^{**19**}**F(281 Mhz):** -151,13 (d, J=22 Hz)

### 15. Synthese der ionische Flüssigkeit 1-Butyl-3-methy-imidazoliumtetrafluoroborat mit nachträglicher Zugabe des Alkalisalzes

In einen 1 I Schlenkkolben werden 114,5 g (1,39 mol) 1-Methylimidazol, 258,1 g (2,79 mol) Butylchlorid gegeben und bei 70°C ca. 3 Tage gerührt. Nach der Bildung von zwei Phasen werden dann zu diesem Gemisch 228,9 g (2,09 mol) Natriumtetrafluoroborat gegeben und bei 70°C für ca. 2 Wochen gerührt. Der entstandene weiße Feststoff wird über eine Schutzgasfritte abfiltriert und die sich gebildeten zwei Phasen voneinander mit Hilfe eines Scheidetrichters getrennt. Die untere gelbliche Phase wird über Nacht bei 60°C im HV getrocknet. Man erhält 1-Butyl-3-methyl-imidazolium tetrafluoroborat in 89 %iger Ausbeute. Zum qualitativen Nachweis auf Chloridreste, werden ca. 1 ml des Produktes mit ca. 5 ml Wasser versetzt und mit 2 Tropfen konzentrierter Salpetersäure angesäuert. Zu dieser Lösung werden dann ca. 3-4 Tropfen Silbernitrat gegeben um etwaig vorhandenes Chlorid als Silberchlorid auszufällen. Das Ausbleiben eines Niederschlags spricht für die komplette Abwesenheit von Chloridresten. 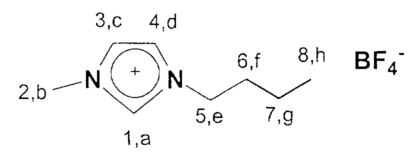 ^{**1**}**H-NMR (300 MHz, CDCl**_{**3**}**):** 0,79 (3 H, tr, J=5,8 Hz, Hₐ); 1,22 (2 H, m, H_{b}); 1,74 (2 H, m, H_{c}); 3,86 (3 H, s, Hₕ); 4,17 (2 H, tr, J=5,8 Hz, H_{d}); 7,51; 7,57 (je 1 H, s, H_{f,g}); 8,7 (1 H, s, Hₑ).
^{**13**}**C-NMR (75 MHz, CDCl**_{**3**}**):** 14,1 (a); 20,3-36,8 (b, c, d); 50,4 (h); 123,2-125,0 (f,g); 137,8 (e).
^{**19**}**F-NMR (281 MHz, CDCl**_{**3**}**):** 148,5-150,8 (m)

### 16. Synthese der ionische Flüssigkeit 1-Butyl-3-methy-imidazoliumtetrafluoroborat mit zusätzlichem Lösungsmittel

In einen 2 I Schlenkkolben werden rund 500 ml frisch destilliertes und über P₂O₅ getrocknetes Aceton vorgelegt und nacheinander 114,5 g (1,39 mol) 1-Methylimidazol, 258,1 g (2,79 mol) Butylchlorid und 228,9 g (2,09 mol) Natriumtetrafluoroborat dazu gegeben und unter Rückfluß für 2 Wochen gerührt. Der entstandene weiße Feststoff wird über eine Schutzgasfritte abfiltriert und die Lösung am Rotationsverdampfer vom Lösungsmittel entfernt. Die überbleibende gelbliche Produktphase wird über Nacht bei 60°C im HV getrocknet. Man erhält 1-Butyl-3-methylimidazoliumtetrafluoroborat in 86 %iger Ausbeute. Zum qualitativen Nachweis auf Chloridreste, werden ca. 1 ml des Produktes mit ca. 5 ml Wasser versetzt und mit 2 Tropfen konzentrierter Salpetersäure angesäuert. Zu dieser Lösung werden dann ca. 3-4 Tropfen Silbernitrat gegeben um etwaig vorhandenes Chlorid als Silberchlorid auszufällen. Das Ausbleiben eines Niederschlags spricht für die komplette Abwesenheit von Chloridresten. 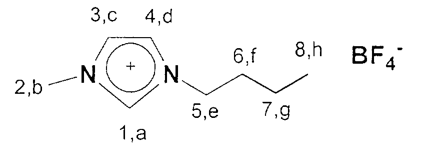 ^{**1**}**H-NMR (300 MHz, CDCl**_{**3**}**):** 0,79 (3 H, tr, J=5,8 Hz, Hₐ); 1,22 (2 H, m, H_{b}); 1,74 (2 H, m, H_{c}); 3,86 (3 H, s, Hₕ); 4,17 (2 H, tr, J=5,8 Hz, H_{d}); 7,51; 7,57 (je 1 H, s, H_{f,g}); 8,7 (1 H, s, Hₑ).
^{**13**}**C-NMR (75 MHz, CDCl**_{**3**}**):** 14,1 (a); 20,3-36,8 (b, c, d); 50,4 (h); 123,2-125,0 (f,g); 137,8 (e).
^{**19**}**F-NMR (281 MHz, CDCl**_{**3**}**):** 148,5-150,8 (m)

## Patentansprüche

1. Verfahren zur Herstellung ionischer Flüssigkeiten der allgemeinen Formel
[A]ₙ⁺[Y]ⁿ⁻,
wobei n = 1 oder 2 ist und
das Anion [Y]ⁿ⁻ ausgewählt ist aus der Gruppe bestehend aus Tetrafluoroborat ([BF₄]⁻), Tetrachloroborat ([BCl₄]⁻), Hexafluorophosphat ([PF₆]⁻), Hexafluoroantimonat ([SbF₆]⁻), Hexafluoroarsenat ([AsF₆]⁻), Tetrachloroaluminat ([AlCl₄]⁻), Trichlorozinkat [(ZnCl₃]⁻), Dichlorocuprat ([CuCl₂]⁻), Sulfat ([SO₄]²⁻ ), Carbonat ([CO₃]²⁻), Fluorosulfonat, [R'-COO]⁻, [R'-SO₃]⁻ oder [(R'-SO₂)₂N]⁻, und R' ein linearer oder verzweigter 1 bis 12 Kohlenstoffatome enthaltender aliphatischer oder alicyclischer Alkyl- oder ein C₅-C₁₈-Aryl-, C₅-C₁₈-Aryl-C₁-C₆-alkyl- oder C₁-C₆-Alkyl-C₅-C₁₈-aryl-Rest ist, der durch Halogenatome substituiert sein kann,
das Kation [A]⁺ ist ausgewählt aus
- quarternären Ammonium-Kationen der allgemeinen Formel
[NR¹R²R³R]⁺,
- Phosphonium-Kationen der allgemeinen Formel
[PR¹R²R³R]⁺,
- Imidazolium-Kationen der allgemeinen Formel 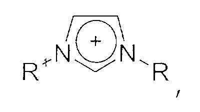 wobei der Imidazol-Kern substituiert sein kann mit wenigstens einer Gruppe, die ausgewählt ist aus C₁-C₆-Alkyl-, C₁-C₆-Alkoxy-, C₁-C₆-Aminoalkyl-, C₅-C₁₂-Aryl- oder C₅-C₁₂-Aryl-C₁-C₆-Alkylgruppen,
- Pyridinium-Kationen der allgemeinen Formel 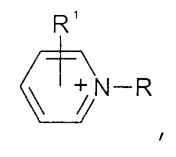 wobei der Pyridin-Kern substituiert sein kann mit mit wenigstens einer Gruppe, die ausgewählt ist aus C₁-C₆-Alkyl-, C₁-C₆-Alkoxy-, C₁-C₆-Aminoalkyl-, C₅-C₁₂-Aryl- oder C₅-C₁₂-Aryl-C₁-C₆-Alkylgruppen,
- Pyrazolium-Kationen der allgemeinen Formel 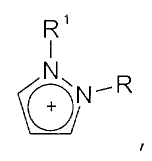 wobei der Pyrazol-Kern substituiert sein kann mit mit wenigstens einer Gruppe, die ausgewählt ist aus C₁-C₆-Alkyl-, C₁-C₆-Alkoxy-, C₁-C₆-Aminoalkyl-, C₅-C₁₂-Aryl- oder C₅-C₁₂-Aryl-C₁-C₆-Alkylgruppen,
- und Triazolium-Kationen der allgemeinen Formel 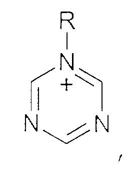 wobei der Triazol-Kern substituiert sein kann mit mit wenigstens einer Gruppe, die ausgewählt ist aus C₁-C₆-Alkyl-, C₁-C₆-Alkoxy-, C₁-C₆-Aminoalkyl-, C₅-C₁₂-Aryl- oder C₅-C₁₂-Aryl-C₁-C₆-Alkylgruppen,
und die Reste R¹, R², R³ unabhängig voneinander ausgewählt sind aus der Gruppe bestehend aus
- Wasserstoff;
- linearen oder verzweigten, gesättigten oder ungesättigten, aliphatischen oder alicyclischen Alkylgruppen mit 1 bis 20 Kohlenstoffatomen;
- Heteroaryl-, Heteroaryl-C₁-C₆-Alkylgruppen mit 3 bis 8 Kohlenstoffatomen im Heteroaryl-Rest und wenigstens einem Heteroatom ausgewählt aus N, O und S, der mit wenigstens einer Gruppe ausgewählt aus C₁-C₆-Alkylgruppen und/oder Halogenatomen substituiert sein können;
- Aryl-, Aryl-C₁-C₆-Alkylgruppen mit 5 bis 12 Kohlenstoffatomen im Arylrest, die gegebenenfalls mit wenigstens einer C₁-C₆-Alkylgruppen und/oder einem Halogenatomen substituiert sein können;
und der Rest R ausgewählt ist aus
- linearen oder verzweigten, gesättigten oder ungesättigten, aliphatischen oder alicyclischen Alkylgruppen mit 1 bis 20 Kohlenstoffatomen;
- Heteroaryl-C₁-C₆-Alkylgruppen mit 3 bis 8 Kohlenstoffatomen im Arylrest und wenigstens einem Heteroatom ausgewählt aus N, O und S, die mit wenigstens einer C₁-C₆-Alkylgruppen und/oder Halogenatomen substituiert sein können;
- Aryl-C₁-C₆-Alkylgruppen mit 5 bis 12 Kohlenstoffatomen im Arylrest, die gegebenenfalls mit wenigstens einer C₁-C₆-Alkylgruppe und/oder einem Halogenenatomen substituiert sein können, durch Alkylierung der zugrundeliegenden Amine, Phosphine, Imidazole, Pyridine, Triazole und Pyrazole mit einem Halogenid RX und Austausch des Halogenidanions X⁻ durch das oben definierte Anion [Y]⁻ oder [Y]²⁻, **dadurch gekennzeichnet, dass** das Verfahren ohne Isolierung der Zwischenprodukte durchgeführt wird.

2. Verfahren nach Anspruch 1 **dadurch gekennzeichnet, dass** der Anionenaustausch mit Metallsalzen der Formel M^{m+}[Y]⁻ₘ, M₂⁺[Y]²⁻ oder M²⁺[Y]²⁻ erfolgt, wobei die Anionen [Y]⁻ und [Y]²⁻ ausgewählt sind aus der in Anspruch 1 definierten Gruppe und m = 1, 2 oder 3 ist.

3. Verfahren nach Anspruch 2 **dadurch gekennzeichnet, dass** das Metall-Ion M ausgewählt ist aus der Gruppen der Alkali- oder Erdalkalimetalle, Blei oder Silber.

4. Verfahren nach irgendeinem der Ansprüche 1 bis 3, **dadurch gekennzeichnet, dass** die Zugabe des zum Anionenaustausch verwendeten Metallsalzes der Formel M^{m+}[Y]⁻ₘ , M₂⁺[Y]²⁻ oder M²⁺[Y]²⁻ zur Reaktionsmischung zu Beginn oder zu jedem späteren Zeitpunkt erfolgt.

5. Verfahren nach Anspruch 1, **dadurch gekennzeichnet, dass** ein molarer Überschuss des zur Alkylierung eingesetzten Halogenids RX verwendet wird.

6. Verfahren nach Anspruch 5, **dadurch gekennzeichnet, dass** das molare Verhältnis des Halogenids RX zu den zugrundeliegenden Aminen, Phosphinen, Imidazolen-, Triazolen-, Pyrazolen-, oder Pyridinen zwischen 1 und 20 liegt.

7. Verfahren nach irgendeinem der Ansprüche 1 bis 6, **dadurch gekennzeichnet, dass** die Reaktion in Substanz oder unter Zusatz eines organischen Lösungsmittels durchgeführt wird.

8. Verfahren nach Anspruch 6, **dadurch gekennzeichnet, dass** als Lösungsmittel Chlorbenzol, Methylenchlorid, Aceton, Acetonitril, Essigester, Methanol, Ethanol, Toluol oder Gemische dieser organischen Lösungsmittel eingesetzt werden.

9. Verfahren nach Anspruch 1 und 8 **dadurch gekennzeichnet, dass** das molare Verhältnis des zugesetzten organischen Lösungsmittels zu den zugrundeliegenden Aminen, Phosphinen, Imidazolen, Triazolen, Pyrazolen oder Pyridinen zwischen 0,5 und 20 beträgt.

10. Verfahren nach irgendeinem der Ansprüche 1 bis 9, **dadurch gekennzeichnet, dass** die Reaktion bei einer Temperatur zwischen -10 und 250°C durchgeführt wird.

11. Verfahren nach irgendeinem der Ansprüche 1 bis 10, **dadurch gekennzeichnet, dass** die Reaktionszeit 5 min bis 300 h beträgt.

12. Verfahren nach irgendeinem der Ansprüche 1 bis 11, **dadurch gekennzeichnet, dass** die ionische Flüssigkeit durch Abfiltrieren des gebildeten festen Metallhalogenids MX isoliert wird.
